# EUROPEAN PATENT APPLICATION

(11) **EP 1 484 603 A1**
(43) Date of publication of application: **08.12.2004**
(21) Application number: 03707202.2
(22) Date of filing: 04.03.2003
(51) Int. Cl.: G01N 27/327

(54) **ANALYZER HAVING INFORMATION RECOGNIZING FUNCTION, ANALYTIC TOOL FOR USE THEREIN, AND UNIT OF ANALYZER AND ANALYTIC TOOL**

(30) Priority: 08.03.2002 JP 2002063762; 08.03.2002 JP 2002063763
(71) Applicant: ARKRAY, Inc., Kyoto-shi, Kyoto 601-8045 (JP)
(72) Inventor: KOBAYASHI, Taizo, c/o ARKRAY, Inc., Kyoto-shi, Kyoto 601-8045 (JP)
(74) Representative: Pluckrose, Anthony William
(86) International application number: PCT/JP2003/002522
(87) International publication number: WO 2003/076918

(57) **Abstract**

The present invention relates to an analyzer (1A) used with an analyzing article (2A) attached thereto, for analysis of a specific component in a sample liquid supplied to the analyzing article (2A). The analyzer (1A) includes an information recognizer for recognition of information added to the analyzing article.. The information recognizer includes an electro-physical-quantity variable part (190A) which has different electro-physical quantities in accordance with the information added to the analyzing article (2A) when the analyzing article (2A) is attached. The analyzing article (2A) includes an information carrier (29A) for giving information to the analyzer (1A). The information carrier (29A) provided by a projection or a hole related to the information to be recognized by the analyzer (1A).

## Description

### TECHNICAL FIELD

The present invention relates to the technology for analyzing a specific component in a sample. More specifically, the present invention relates to an analyzing article used in analysis of a sample, an analyzer, and a unit of the analyzing article and the analyzer.

### BACKGROUND ART

As a common method of measuring a specific component in the body fluid such as glucose in the blood, an oxidation-reduction reaction promoted by an oxidation-reduction enzyme is utilized. On the other hand, for handy measurement of the blood sugar levels at home and elsewhere, palm-size, portable blood sugar level testers are used widely. These handy-type blood sugar level testers make use of disposable biosensors, which also provides an enzyme reaction field. The blood sugar level measurement is made by supplying the blood to the biosensor.

Sensitivity of the individual biosensors can vary from one biosensor to another. The variation can be a result of difference in row materials, design changes in production lines and so on. Especially, when starting up the production line, due to needs for optimizing various conditions in the production line and selecting suitable materials, sensitivity variations among the produced sensors tend to be large. Further, when there are plural manufacturing plants or plural production lines of the biosensors, sensitivity variation among the plants or production lines can result. In preparation for these sensitivity variations, some blood sugar level testers incorporate a plurality of calibration curves. In addition, if the tester is capable of not only measuring the blood sugar level but also other values such as cholesterol level, a plurality of calibration curves must be prepared so that each kind of the components can be measured.

In these cases, arrangements must be made so that the measuring device can recognize information necessary for relating the calibration curve with the biosensor or target component. An example of such arrangements is disclosed in JP-A 10-332626. According to the invention disclosed in the gazette, the biosensor is provided with production-lot identifying electrodes separately from concentration-level measuring electrodes, and the biosensor outputs signals unique to the locations of the production-lot identifying electrodes. The measuring device on the other hand has a plurality of detecting terminals corresponding to the production-lot identifying electrodes. The measuring device uses these detecting terminals to pick up the signals unique to the locations of the production-lot identifying electrodes, so that the blood sugar level tester can choose an appropriate calibration curve based on the signals obtained trom the biosensor.

However, the invention disclosed in the above gazette has the following problems:

A first problem relates to manufacturing of the biosensors due to an arrangement that the production-lot identifying electrodes are formed on the same side of a substrate on which measuring electrodes are formed. When embodying this arrangement, the measuring electrodes and the production-lot identifying electrodes may be formed simultaneously by screen printing, vapor depositing and so on. A challenge in this case is that the sensitivity of the biosensor must be forecasted and the production-lot identifying electrodes must be formed on the forecast. If a large discrepancy is found between the actual sensor sensitivity and the forecast sensitivity, the produced biosensors must be scrapped, resulting in decreased yield. If the measuring electrodes and the production-lot identifying electrodes are formed in separate steps, then the steps for forming the production-lot identifying electrodes are extra steps involving complex operations such as screen printing or vapor depositing, which will decrease operation efficiency.

A second problem relates to construction. Specifically, when the biosensor is attached to the measuring device, the production-lot identifying electrodes of the biosensor make contact with the counterpart terminals of the measuring device. This contact is made every time the biosensor is attached to the measuring device, making the identification terminals prone to deterioration. If the identification terminals are prone to deterioration, the measuring device must be repaired or serviced frequently, or the deterioration itself shortens the life of the device.

A third problem relates to the measuring device itself, due to the fact that the production-lot identifying electrodes are formed on the same side of the substrate on which the measuring electrodes are formed. In order to accept this type of biosensor, the measuring device has to have its measuring electrodes and a corresponding number of the production-lot identifying terminals disposed on a single side of a substrate. This means that all of these electrodes must be disposed in a very small area under very tight limitations. This poses significant limitations in designing the part of measuring device to which the biosensor is attached, decreasing freedom in design. Such a problem becomes more conspicuous as the number of identifying terminals increases with increasing amount of information to be recognized by the measuring device.

### DISCLOSURE OF THE INVENTION

The present invention aims at adding information to an analyzing article for recognition by an analyzer, advantageously in terms of cost.

The present invention also aims at reducing deterioration in part of the analyzer where the information from the analyzing article is recognized.

Further, the present invention aims at enabling the analyzer to recognize the information from the analyzing article without very much sacrificing freedom in the design of the analyzer.

A first aspect of the present invention provides an information recognizing analyzer used with an analyzing article attached thereto. The analyzer analyzes a specific component in a sample liquid supplied to the analyzing article, and comprises an information recognizer which recognizes information added to the analyzing article. The information recognizer includes an electro-physical-quantity variable part which has different electro-physical quantities in accordance with the information added to the analyzing article upon attachment of the analyzing article.

The electro-physical-quantity variable part includes for example, a pair of a first electrode and a second electrode in a relative positional relationship which is variable upon attachment of the analyzing article.

The first electrode and the second electrode have their distance between the two varied, for example.

At least one of the first electrode and the second electrode in the information recognizer further includes a fixed elastic member. In this case, preferably, the electro-physical-quantity variable part has the distance between the first electrode and the second electrode varied by an elastic deformation of the elastic member.

The first electrode and the second electrode may have their area of mutually opposed surfaces varied.

At least one of the first electrode and the second electrode in the information recognizer moves upon attachment of the analyzing article, in a direction of insertion of the analyzing article.

The information recognizer may include pairs of electrodes each provided by a first electrode and a second electrode in a relative positional relationship which is variable upon attachment of the analyzing article. In this case, preferably, information is recognized individually from each pair of electrodes.

Preferably, the information recognizer further includes a capacity measurer which measures a capacity of a capacitor constituted by the first electrode and the second electrode, and an information calculator which recognizes information added to the analyzing article based upon a comparison between a result of measurement obtained by the capacity measurer and a predetermined threshold value.

The electro-physical-quantity variable part may include a pressure sensitive electric conductor which has a resistance value variable upon attachment of the analyzing article.

The electro-physical-quantity variable part may include a plurality of pressure sensitive electric conductors which have a resistance value variable upon attachment of the analyzing article. In this case, information may be recognized individually from each pressure sensitive electric conductor.

The information recognizer may further include a resistance value measurer which measures a resistance value of the pressure sensitive electric conductor, and an information calculator which recognizes information added to the analyzing article based upon a comparison between a result of measurement obtained by the resistance value measurer and a predetermined threshold value.

A second aspect of the present invention provides an information recognizing analyzer used with an analyzing article attached thereto, for analysis of a specific component in a sample liquid supplied to the analyzing article. The analyzer comprises an information recognizer which recognizes information added to the analyzing article. The information recognizer includes a first and a second conductors, which make or do not make a mutual contact in accordance with the information added to the analyzing article upon attachment of the analyzing article.

The information recognizer may further include a contact detector which detects a contact between the first conductor and the second conductor, and an information calculator which recognizes information added to the analyzing article based upon a result of detection obtained by the contact detector.

A third aspect of the present invention provides an analyzing article comprising an information carrier which gives information to an analyzer. The analyzer comprises an information recognizer which recognizes the information carried by the information carrier. The analyzing article is used as attached to the analyzer. The information recognizer includes an electro-physical-quantity variable part which has different electro-physical quantities upon attachment of the analyzing article. The information carrier is provided by a projection or a hole related to the information to be recognized by the analyzer.

The analyzing article is formed essentially into a platy shape for example. In this case, the projection or the hole projects or recesses in a direction generally perpendicular to a thickness direction of the analyzing article. Alternatively, the projection or the hole may projects or recesses in a thickness direction of the analyzing article.

The projection or the hole projects or recesses by an amount related to the information to be recognized by the analyzer.

The hole may be provided by a through-hole.

A fourth aspect of the present invention provides a unit of an analyzing article and an analyzer for analysis of a specific component in a sample liquid supplied to the analyzing article. The analyzer includes a first electrode fixed therein whereas the analyzing article includes a second electrode fixed therein so as to face the first electrode and thereby constitute a capacitor upon attachment of the analyzing article to the analyzer.

The analyzer may further include a capacity measurer which measures a capacity of the capacitor constituted by the first electrode and the second electrode, and an information calculator which recognizes information added to the analyzing article based upon a comparison between a result of measurement obtained by the capacity measurer and a predetermined threshold value.

### BRIEF DESCRIPTION OF THE DRAWINGS

Fig. 1 illustrates a first embodiment of the present invention, showing an analyzer in a block diagram and a biosensor in a plan view.
Fig. 2 is an overall perspective view of the biosensor in Fig. 1.
Fig. 3 is an exploded perspective view of the biosensor in Fig. 2.
Fig. 4 gives plan views, showing different kinds of an information carrier formed in the biosensor.
Fig. 5 is a sectional view taken in lines V-V in Fig. 1.
Fig. 6 is a conceptual diagram of an information recognizer.
Fig. 7 is a sectional view showing a primary portion of the analyzer, for describing a layout example of capacity sensors.
Fig. 8 is an exploded perspective view, showing a partial cutout of the capacity sensor.
Fig. 9 is a sectional view, showing an enlarged view around the capacity sensors.
Fig. 10 is a sectional view, showing a primary portion of the analyzer for describing another layout example of the capacity sensors.
Fig. 11 is an overall perspective view as seen from a back side, of a biosensor according to a second embodiment of the present invention.
Fig. 12 is a sectional view of a primary portion of the analyzer according to the second embodiment of the present invention, loaded with a biosensor.
Fig. 13 shows a third embodiment of the present invention in a sectional view of a primary portion of an analyzer loaded with a biosensor.
Fig. 14 is an overall perspective view of a biosensor according to a fourth embodiment of the present invention.
Fig. 15 is a conceptual diagram of an information recognizer in an analyzer according to the fourth embodiment of the present invention.
Fig. 16 is a sectional view of a primary portion of the analyzer according to the fourth embodiment, loaded with the biosensor.
Fig. 17 shows a fifth embodiment of the present invention in a sectional view of a primary portion of an analyzer loaded with a biosensor.
Fig. 18 is a conceptual diagram of an information recognizer of the analyzer in Fig. 17.
Fig. 19 is a sectional view showing a primary portion of the analyzer, for describing a layout example of information recognition elements in the information recognizer in Fig. 18.
Fig. 20 is a sectional view, showing an enlarged view around the information recognition elements in the analyzer in Fig. 17.
Fig. 21 is a sectional view showing a primary portion of the analyzer, for describing another layout example of the information recognition elements.
Fig. 22 is a sectional view showing a primary portion of the analyzer, for describing another information recognition element.
Fig. 23 is a sectional view of a primary portion of an analyzer according to a sixth embodiment of the present invention, loaded with a biosensor.
Fig. 24 illustrates a seventh embodiment of the present invention, showing an analyzer in a block diagram and a primary portion of a biosensor in a plan view.
Fig. 25 is an overall perspective view of the biosensor in Fig. 25.
Fig. 26 gives plan views, showing different kinds of an information carrier formed in the biosensor in Fig. 24.
Fig. 27 is a sectional view of a primary portion of the analyzer according to the seventh embodiment of the present invention, loaded with a biosensor.

### BEST MODE FOR CARRYING OUT THE INVENTION

First, a first embodiment will be described.

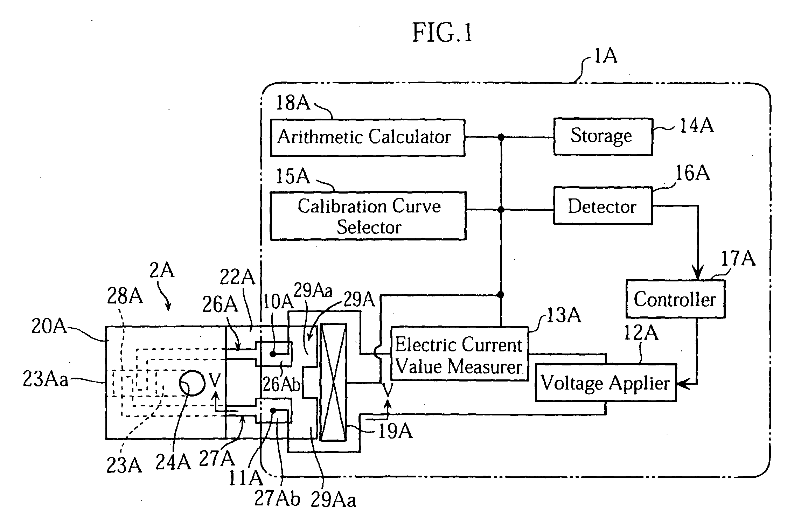

As shown in Fig. 1, an analyzer 1A uses a biosensor 2A. The analyzer 1A is capable of performing an electrochemical measurement on the concentration of a specific component in a sample fluid supplied to the biosensor 2A.

The analyzer 1A generally includes measuring terminals 10A, 11A, a voltage applier 12A, an electric current value measurer 13A, a storage 14A, a calibration curve selector 15A, a detector 16A, a controller 17A, an arithmetic calculator 18A and an information recognizer 19A. Details of the components 10A-19A will be described later.

The biosensor 2A, on the other hand, includes a cover 20A, a spacer 21A and a substrate 22A as clearly shown in Fig. 1 through Fig. 3, and these components provide a passage 23A.

The cover 20A has a hole 24A in order to allow gas in the passage 23A to escape. The spacer 21A has a slit 25A. The slit 25A determines the size of the passage 23A and has an open end 25Aa. The passage 23A communicates with the outside via the open end 25Aa of the slit 25A and the hole 24A. The open end 25Aa serves as a sample liquid inlet 23Aa. According to this construction, a sample liquid supplied through the sample liquid inlet 23Aa moves through the passage 23A toward the hole 24A by capillarity.

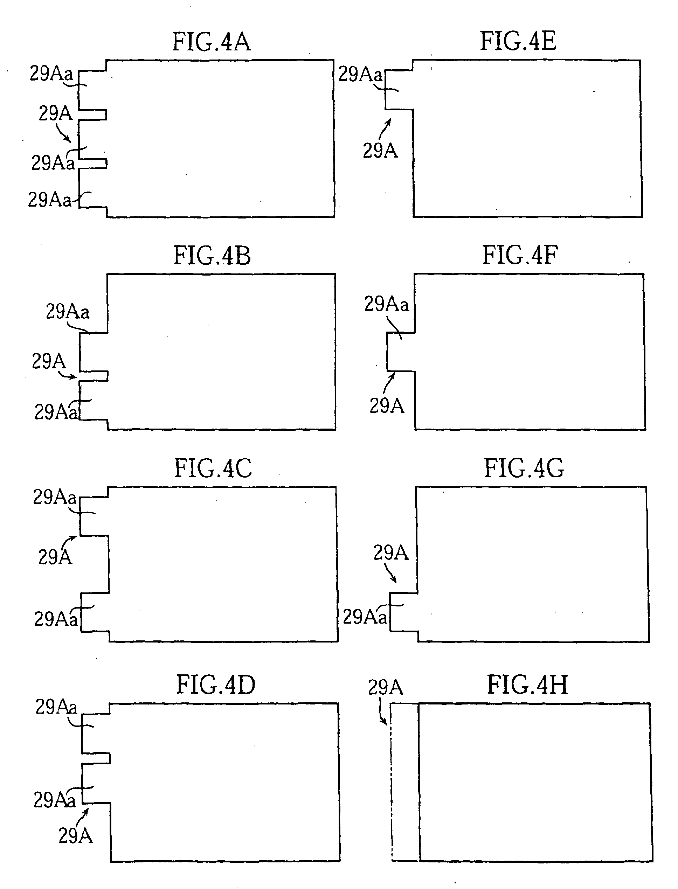

The substrate 22A is generally rectangular, and has an end serving as an information carrier 29A. The information carrier 29A allows the information recognizer 19A of the analyzer 1A to recognize information about e.g. the biosensor 2A. As shown in Fig. 4A through Fig. 4H for example, the information carrier 29A has three predetermined regions. By selecting whether or not to form a projection 29Aa, for each of the three regions, information is assigned for recognition by the information recognizer 19A of the analyzer 1A (See Fig. 1). The information carrier 29A can be formed by punching for example, or other means which are much easier than screen printing or vapor depositing. Therefore, addition of the information carrier 29A to the biosensor 2A does not very much decrease operation efficiency.

The information about the biosensor 2A may include any data necessary for the calibration curve selector 15A to select a calibration curve (correction data), specific information about the biosensor 2A (e.g. date of manufacture, deadline date for use, name of manufacturer, and place of manufacture (country of origin, name of the plant)), and a production lot ID information (LOT number) of the biosensor 2A.

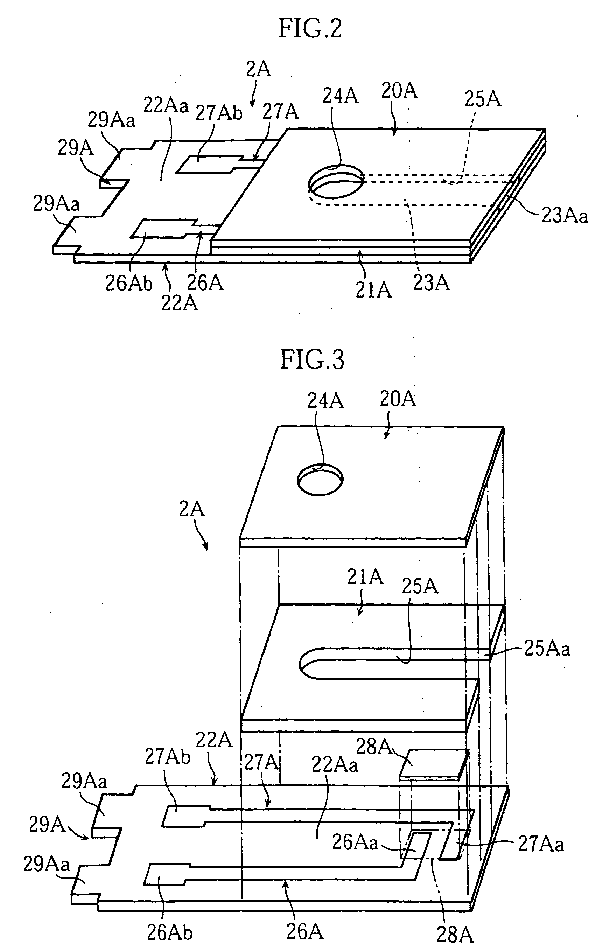

As shown in Fig. 2 and Fig. 3, the substrate 22A has an upper surface 22Aa provided with a reaction electrode 26A, a pairing electrode 27A and a reagent layer 28A.

The reaction electrode 26A and the pairing electrode 27A each extend primarily longitudinally of the substrate 22A, with their ends 26Aa, 27Aa extending widthwise of the substrate 22A. Therefore, the reaction electrode 26A and the pairing electrode 27A each have a shape like a letter L as a whole. The ends 26Ab, 27Aab of the reaction electrode 26A and the pairing electrode 27A serve as terminals for making contact respectively with the terminals 10A, 11A of the analyzer 1A.

The reagent layer 28A is solid for example, bridging across the end 26Aa of the reaction electrode 26A and the end 27Aa of the pairing electrode 27A. The reagent layer 28A is provided by e.g. a mediator (electron transfer material) doped with an oxidation-reduction enzyme, and is dissolved when a sample liquid is introduced to the passage 23A. The reagent layer 28A dissolves to form a liquid reaction system within the passage 23A.

The electron transfer material is provided by e.g. a complex of iron or Ru. The oxidation-reduction enzyme is selected in accordance with a specific component which is a target of measurement. Examples of the specific component include glucose, cholesterol and lactic acid. Examples of the oxidation-reduction enzyme for these specific components include glucose dehydrogenase, glucose oxidase, cholesterol dehydrogenase, cholesterol oxidase, lactic acid dehydrogenase and lactic acid oxidase.

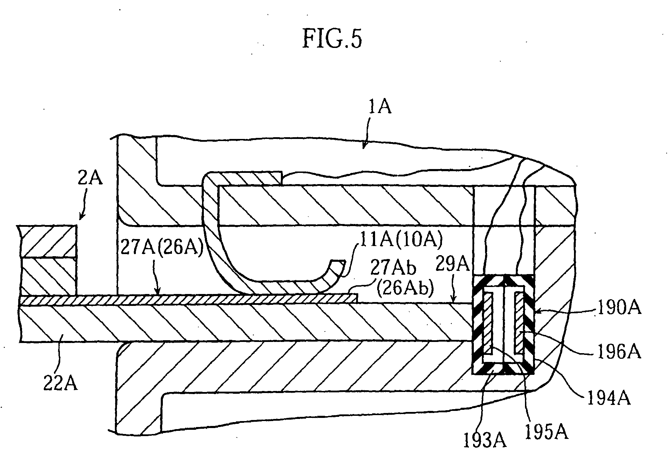

The measuring terminals 10A, 11A of the analyzer 1A in Fig. 1 make contact with the ends 26Ab, 27Ab of the reaction electrode 26A and the pairing electrode 27A as shown in Fig. 5. The measuring terminals 10A, 11A are used e.g. for applying a voltage to the liquid reaction system through the reaction electrode 26A and the pairing electrode 27A, or measuring the amount of electron exchange between the liquid reaction system and the reaction electrode 26A.

The voltage applier 12A in Fig. 1 applies a voltage to the liquid reaction system via the measuring terminals 10A, 11A. The voltage applier 12A is provided by a DC power source such as an ordinary dry battery or a rechargeable battery.

The electric current value measurer 13A measures the value of a current or the amount of electrons supplied to the reaction electrode 26A from the reagent layer 28A for example, when a constant voltage is applied to the reagent layer 28A.

The storage 14A stores data about a plurality of calibration curves.

The calibration curve selector 15A selects an appropriate calibration curve matched with the sensitivity of a given biosensor 2A based on infcrmation supplied from e.g. the information carrier 29A of the biosensor 2A.

The detector 16A detects whether the sample liquid has been introduced into the passage 23A, based on the value of electric current measured at the electric current value measurer 13A.

The controller 17A controls the voltage applier 12A and selects a state where there is an electric potential difference between the reaction electrode 26A and the the concentration level of a specific component in the sample liquid, based on the value of response current measured by the electric current value measurer 13A and the calibration curve selected by the calibration curve selector 15A.

Each of the storage 14A, the calibration curve selector 15A, the detector 16A, the controller 17A and the arithmetic calculator 18A can be provided by a CPU, a ROM, a RAM or a combination thereof. Further, all of these elements can be provided by a single CPU connected with a plurality of memories.

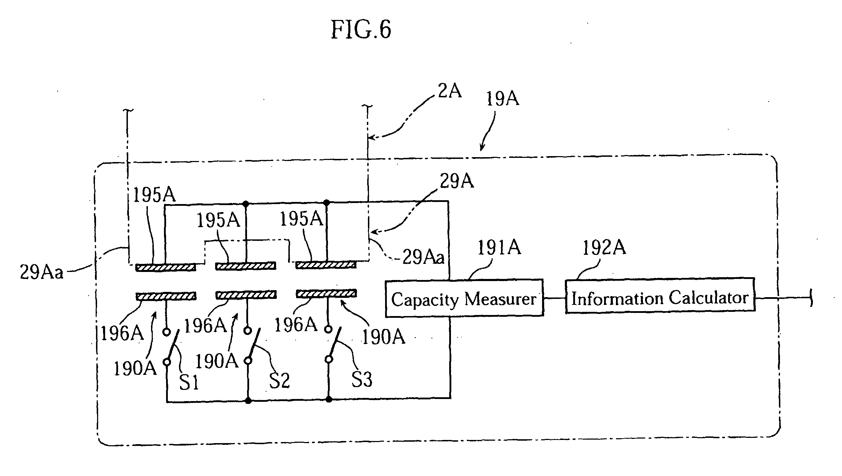

The information recognizer 19A recognizes information added to the biosensor 2A, based on the construction of the information carrier 29A of the biosensor 2A. As shown in Fig. 6, the information recognizer 19A has three capacity sensors 190A, a capacity measurer 191A and an information calculator 192A.

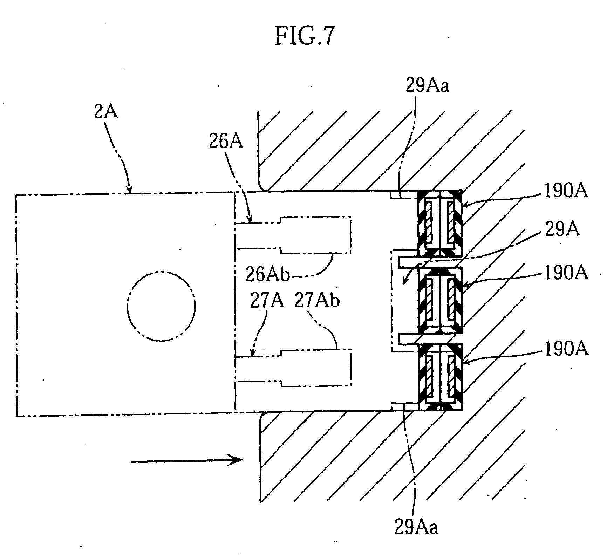

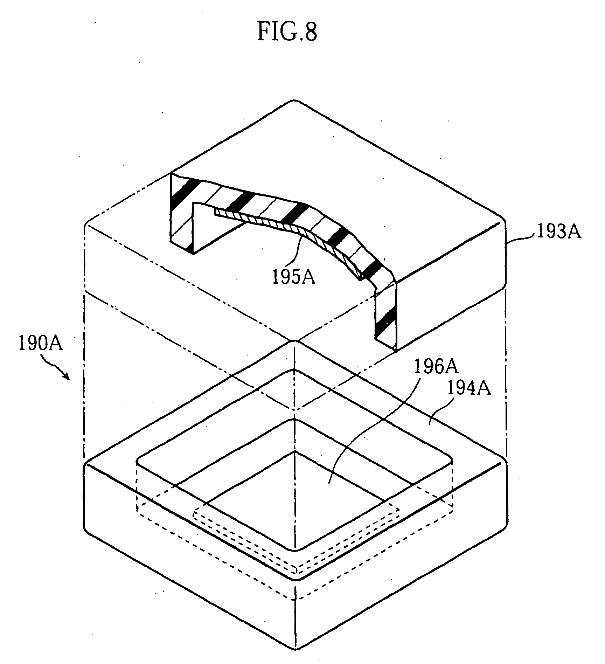

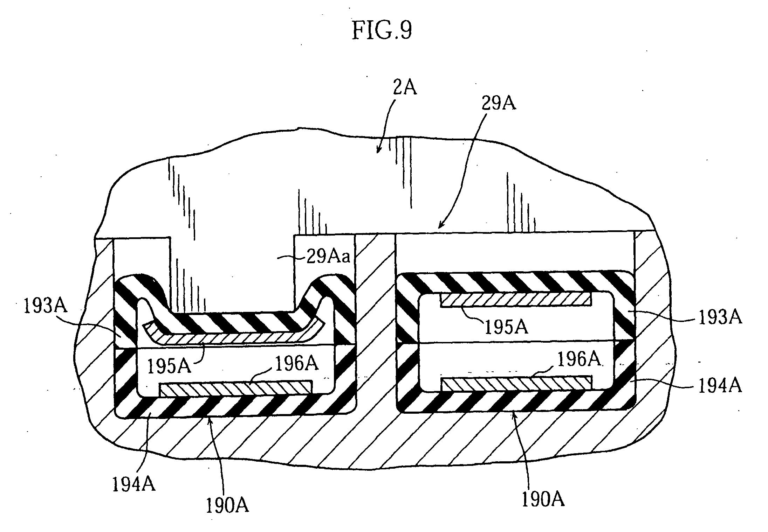

As expected from Fig. 5 and Fig. 7, each capacity sensor 190A is located so that it can be pressed by a corresponding projection 29Aa of the biosensor 2A when the biosensor 2A is attached to the analyzer 1A. As shown in Fig. 8, each capacity sensor 190A is provided by an opposed pair of elastic members 193A, 194A coupled to each other. Each of the elastic members 193A, 194A is like a bottomed box made of rubber for example. Each of the elastic members 193A, 194A has an inner bottom formed with a first or a second electrode 195A or 196A. In other words, the first and the second electrodes 195A, 196A are opposed to each other with a space in between filled with air. The distance between these electrodes 195A, 196A varies as the elastic member 193A (194A) makes elastic deformation, and each capacity sensor 190A provides a variable capacitor whose capacity varies as the distance changes between the first and the second electrodes 195A, 196A. In the analyzer 1A, when the biosensor 2A is attached and the projections 29Aa presses the elastic members 193A as shown in Fig. 9, the distance between the first and the second electrodes 195A, 196A is changed. Specifically, the distance between the first and the second electrodes 195A, 196A becomes smaller, which increases the capacity of the capacity sensor 190A.

As shown in Fig. 6, the capacity measurer 191A is connected with the first and the second electrodes 195A, 196Avia switches S1-S3. Therefore, the capacity measurer 191A can measure the capacity of each capacity sensor 190A individually, by selecting the open/close status of the switches S1-S3.

The information calculator 192A performs calculation to obtain information supplied from the information carrier 29A, based on the capacities of the capacity sensors 190A. The information calculator 192A compares e.g. a result of measurement given by the capacity measurer 191A with a predetermined threshold value determined for eachcapacity measurer 191A, and uses a result of the comparison as the basis of the calculation. The threshold value is, for example, an intermediate value between a capacity value when the capacity sensor 190A is pressed by the projection 29Aa and a capacity value when the sensor is not pressed. Then, a capacity sensor 190A which is pressed by the projection 29Aa and therefore has the distance shortened between its first and second electrodes 195A, 196A is recognized as having a larger capacity (H signal) than the threshold value, by the information calculator 192A. On the other hand, a capacity sensor 190A which is not pressed by the projection 29Aa is recognized as having a smaller capacity (L signal) than the threshold value.

According to the present embodiment, up to three projections 29Aa can be formed in the biosensor 2A (See Fig. 4), and the information recognizer 19A is provided with three capacity sensors 190A (See Fig. 6). This provides a total of eight combinations of the H signal and the L signal obtainable by the information recognizer 19A. Thus, the information recognizer 19A can recognize eight kinds of information individually.

Next, description will be made for a concentration measuring operation by the analyzer 1A. The description assumes that the analyzer 1A is a measuring instrument for measurement of blood glucose level, and that information supplied from the information carrier 29A of the biosensor 2A to the analyzer is information on the sensitivity of the biosensor 2A (information necessary for selecting a calibration curve).

When quantifying the glucose level, first, the user attaches a biosensor 2A to an analyzer 1. Upon the attachment of the biosensor 2A, the information recognizer 19A automatically recognizes correction information for the biosensor 2A. As has been described, the correction information is obtained as a combination of the H signal and the L signal represented by the number and location of the projections 29Aa formed in the information carrier 29A of the biosensor 2A. Upon recognition of the correction information, the calibration curve selector 15A selects a calibration curve appropriate for the attached biosensor 2A from a plurality of calibration curves stored in the storage 14A.

Such an arrangement for automatic selection of the calibration curve eliminates any burden on the part of the user, such as operating a button on the analyzer 1A or attaching a calibration curve correction chip to the analyzer 1A, when selecting the calibration curve. Thus, an appropriate selection of the calibration curve can be made without burden to the user, and reliably in accordance with the sensitivity of each sensor.

After the biosensor 2A is attached, the biosensor 2A is supplied with blood via the sample inlet 23Aa. In the biosensor 2A, the blood is introduced into the passage 23A by capillarity, and the blood dissolves the reagent layer 28A, establishing a liquid reaction system within the passage 23A, and in this liquid reaction system, glucose in the blood is oxidized and the electron transfer material is reduced.

Meanwhile, the voltage applier 12A applies a constant voltage between two ends 26Aa, 27Aa of the reaction electrode 26A and the pairing electrode 27A since before the time the blood is supplied. This voltage application allows the reduced electron transfer material in the liquid reaction system to give electrons to the reaction electrode 26A and thereby to become an oxide. The electric current value measurer 13A measures, at a predetermined time interval, the amount of electrons supplied to the reaction electrode26A, and results of the measurements aremonitored by the detector 16A. The detector 16A also checks if the measured current value has exceeded a predetermined threshold value, and determines that the blood has been introduced to the biosensor 2A once the value has exceeded the threshold value.

The determination is sent to the controller 17A. In response, the controller 17A has the voltage applier 12A stop the voltage application. The electric current value measurer 13A continues the measurement of current value at the predetermined time interval even after the voltage application to the liquid reaction system is stopped. Once the voltage application to the liquid reaction system is stopped, the electron transfer material in the reduced form begins to accumulate in the liquid reaction system. When a predetermined time has been passed since the stoppage of voltage application, the controller 17A has the voltage applier 12A apply a voltage again to the liquid reaction system. The arithmetic calculator 18A obtains a current value measured after a predetermined time has been passed since the resumption of the voltage application to the liquid reaction system, and this value is used as a response current value for calculation. The arithmetic calculator 18A calculates the blood glucose level based on the obtained response current value and the given calibration curve. Alternatively, the response current value may be converted to a voltage value, and the calculation of the blood glucose level is based on the voltage value and the calibration curve.

According to the present embodiment, as shown in Fig. 9, attachment of the biosensor 2A changes positional relationship between the first and the second electrodes 195A, 196A of the information recognizer 19A, through which the analyzer 1A recognizes information added to the biosensor 2A. Inotherwords, there is no need for providing the biosensor 2A with production-lot identifying electrodes, and accordingly there no longer is need for providing the analyzer 1A with production-lot identifying terminals. Further, when recognizing information, the biosensor 2A may not necessarily contact with the first and the second electrodes 195A, 196A of the information recognizer 19A. There is no need either for the first and the second electrodes 195A, 196A to make contact with each other since they constitute a capacitor. Therefore, even if the analyzer 1A is loaded with a biosensor 2A for a multiple of times, the first and the second electrodes 195A, 196A do not deteriorate easily, and as a result, essential portions in recognizing information from the biosensor 2A do not deteriorate easily, eliminating needs for frequent repair or maintenance even if the attachment of the biosensor 2A is made for a multiple of times.

In the description of the above embodiment, the information recognizer 19A of the analyzer 1A has three capacity sensors 190A. However, the number of the capacity sensors may not be three. The quantity of the capacity sensors may be selected in accordance with types of information to be re cognized by the information recognizer .

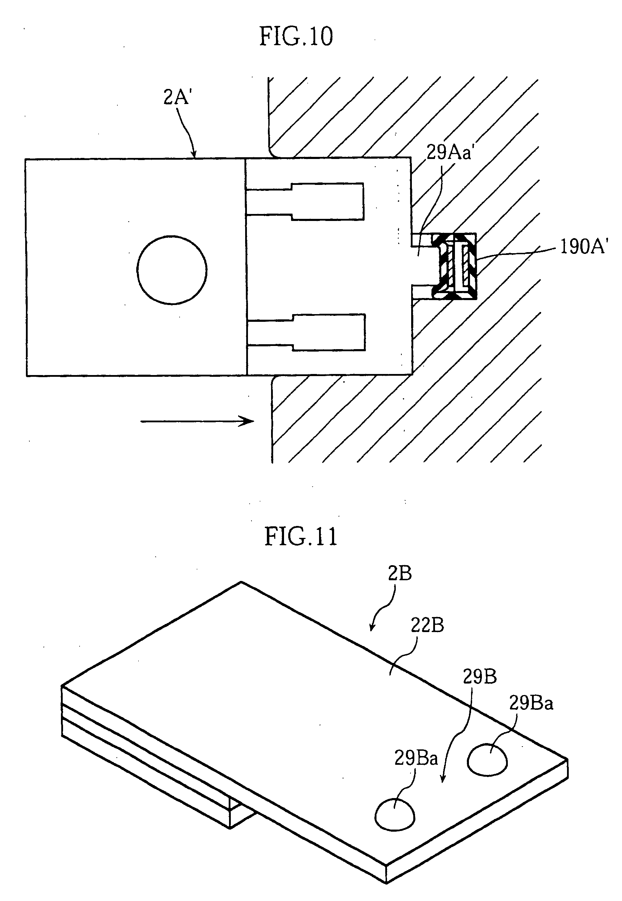

The information recognizer may be used, in addition to recognizing information relevant to the biosensor, to recognize that the biosensor has been attached for example. Such an attachment recognition can be achieved as shown in Fig. 10 by providing a biosensor 2A' with an attachment recognition projection 29Aa' and disposing a capacity sensor 190A' at a location corresponding to the projection 29Aa'. In this arrangement, attachment of the biosensor 2A' causes the projection 29Aa' to press the capacity sensor 190A', thereby giving an H signal, whereas an L signal is given if the biosensor 2A' is not attached. Thus, the attachment of the biosensor 2A' can be recognized when the H signal is obtained. With this arrangement, the information recognizer may turn on the main power for example, upon reception of the H signal.

The information recognizer may have a plurality of capacity sensors, one of which may be used to turn on the main power, with the rest to recognize the information about the biosensor. For example, in the analyzer 1A in Fig. 1, one capacity sensor 190A of the three capacity sensors 190A (See Fig. 6, Fig. 7 and so on.) may be used as a sensor for detecting attachment of the biosensor (thereby turning on the main power). With this arrangement, the remaining two capacity sensors 190A are used for information recognition for selecting a calibration curve.

In the embodiment described above, a two-level signal or an H signal and an L signal are obtained from each of the capacity sensors. Alternatively, the signal may have three or more levels. For example, the projection of the biosensor may have a specific length so that the distance between the first and the second electrodes of the capacity sensor will have a plurality of values representing a plurality of levels of the H signal outputted from the capacity sensor.

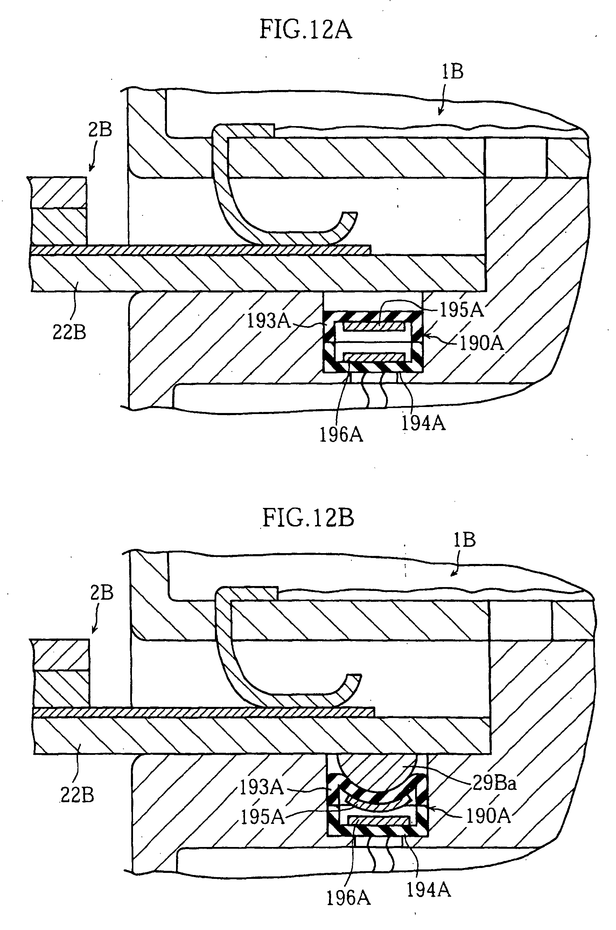

Next, a second embodiment of the present invention will be described with reference to Fig. 11 as well as Fig. 12A and Fig. 12B.

In a biosensor 2B in Fig. 11, a quantity (including zero) of projections 29Bb and locations of the projections 29Bb represent information to be recognized by an analyzer 1B in Fig. 12A and Fig. 12B. The projections 29Bb are hemispheres projecting from a back surface of a substrate 22B. Projections such as the projections 29Bb offer advantages, for example, that the user can easily tell the top and the back surfaces of the biosensor 2B, and the biosensor 2B can be easily picked or removed when it is placed on a flat surface like a table.

The projections 29Bb can be formed, for example, by first preparing a thermoplastic resin in a molten or a pasty form softened with a solvent, potting and then allowing the resin to set on the back surface of the substrate 22B. Such an operation is significantly easier than screen printing or vapor depositing, and thus the addition of the information carrier 29B (projections 29Bb) to the biosensor 2B can be achieved without very much decreasing efficiency in manufacture. The projections 29Bb may not be hemispheres.

On the other hand, as clearly shown in Fig. 12A and Fig. 12B, the analyzer 1B uses capacity sensors 190A which are similar to those used in the previous embodiment (See Fig. 8). When the biosensor 2B is attached to the analyzer 1B, the projections 29Bb press the capacity sensors 190A. A quantity of the capacity sensors 190A is three for example, being equal to a maximum number of the projections 29Bb which can be formed.

According to this arrangement, as shown in Fig. 12B, when a projection 29Bb of the biosensor 2B presses the corresponding capacity sensor 190A, there is a change in the distance between a first and a second electrodes 195A, 196A of the capacity sensor 190A in directions of thickness of the biosensor 2B. The change in the distance between the first and the second electrodes 195A, 196A causes the capacity sensor 190A to output an H signal. On the other hand, as shown in Fig. 12A, a capacity sensor 190A which is not pressed by a projection 29Bb and therefore has no change in the distance between its first and second electrodes 195A, 196A outputs an L signal. Thus, the information recognizer (not illustrated) can recognize information from the information carrier 29B by using the same method as described for the previous embodiment.

The second embodiment allows the same design variations as the first embodiment.

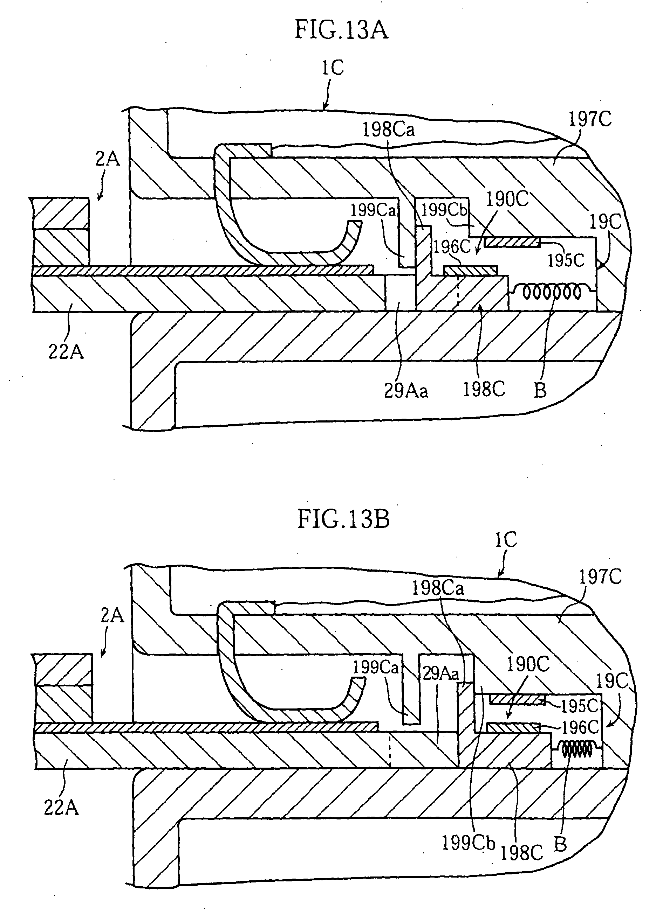

Next, a third embodiment of the present invention will be described with reference to Fig. 13A and Fig. 13B.

A biosensor 2A shown in Fig. 13A and Fig. 13B is the same as the one used in the first embodiment (See Fig. 2 through Fig. 4). Specifically, the biosensor 2A includes a substrate 22A having an end having specific regions where projections 29Aa can be made. By selecting whether to form a projection 29Aa or not, for each of the three regions, information is assigned for recognition by the analyzer 1C.

On the other hand, the analyzer 1C shown in the same figures differs from those used in the first and the second embodiments, in the construction of information recognizer 19C. Though not illustrated very much clearly in the Figures, the information recognizer 19C has three capacity sensors 190C. Each capacity sensor 190C has a first and a second electrodes 195C, 196C, and the first and the second electrodes 195C, 196C can make a relative movement to each other in a direction in which the biosensor 2A is inserted. Specifically, the capacity sensor 190C has its first and second electrodes 195C, 196C faced to each other so that the area of opposed regions (the area where the first and the second electrodes 195C, 196C overlap each other) is variable and so its capacity is variable.

More specifically, the first electrode 195C is unmovably fixed to a casing 197 of the analyzer 1C whereas the second electrode 196C is fixed to a slider 198C and is movable in the direction of insertion of the biosensor 2A. The slider 198C has an interferer 198Ca. As shown clearly in Fig. 13A, the interferer 198Ca is normally urged by a spring B onto a first stopper 199Ca of the casing 197. Under this state, the opposed area of the first and the second electrodes 195C, 196C is small (the opposed area may be zero), and the capacity sensor 190C outputs an L signal. On the other hand, when a force is applied to the slider 198C in the right hand direction (in the inserting direction of the biosensor 2A), the slider 198C is moved, as understood from Fig. 13B, within a range until the interferer 198Ca interferes with a second stopper 199Cb. As the slider 198C or the second electrode 196C is moved, the opposed area of the first and the second electrodes 195C, 196C increases from the normal state, enabling the capacity sensor 190C to output an H signal.

As has been mentioned earlier, the biosensor 2A according to the present embodiment is the same as the one used in the first embodiment. Therefore, when the biosensor 2A is attached to the analyzer 1C, it is possible to cause the projections 29Aa of the biosensor 2A to move the slider 198C or the second electrode 196C. In other words, by selecting locations and a quantity of the projections 29Aa in the biosensor 2A, it is possible to have each of the capacity sensors 190C output the H signal or the L signal individually.

The second electrode 196C may not necessarily be fixed onto the slider 198C. For example, the second electrode may be formed of a platy member having a sufficient strength so that the second electrode is directly moved by the projection of the biosensor.

The capacity sensors may be able to obtain three or more levels of the signal. For example, the projection of the biosensor may have a specific length so that the distance between the first and the second electrodes of the capacity sensor will have a plurality of values representing a plurality of levels of the H signal outputted from the capacity sensor. As another variation, the capacity sensor may have the area of opposed surfaces of the first and the second electrodes decreased when the second electrode is moved relatively by the projection of the biosensor.

The information recognizer may have more than three capacity sensors, so that the information recognizer will not only recognize information relevant to the biosensor but also recognize that the biosensor has been attached for example, or turns on the main power of the analyzer upon recognition of the information.

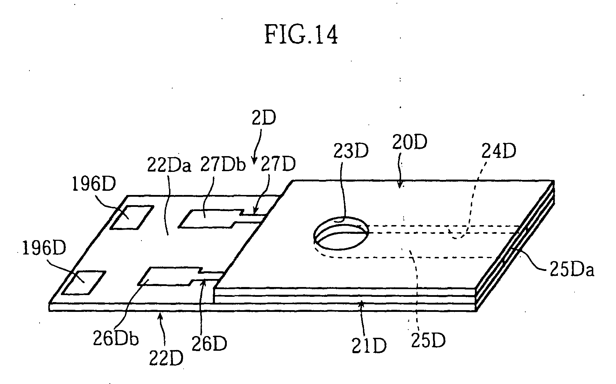

Next, a fourth embodiment of the present invention will be described with reference to Fig. 14 through Fig. 16. Throughout these figures, members and components already shown in the previous figures will be identified with the same alpha-numerical codes and will not be described here again.

A biosensor 2D in Fig. 14 includes a substrate 22Dhaving an end including specific regions. For each of the regions an electrode 196D can be formed. By making a selection whether to form or not to form the electrode 196D, for each location, information is assigned for recognition by an analyzer 1D.

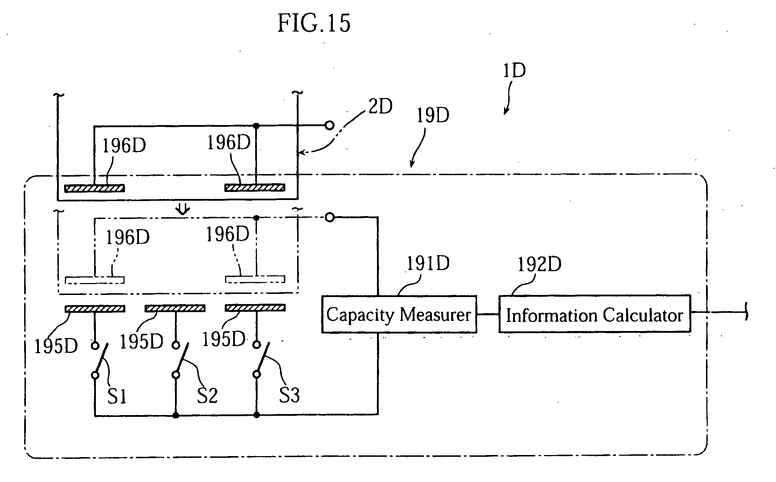

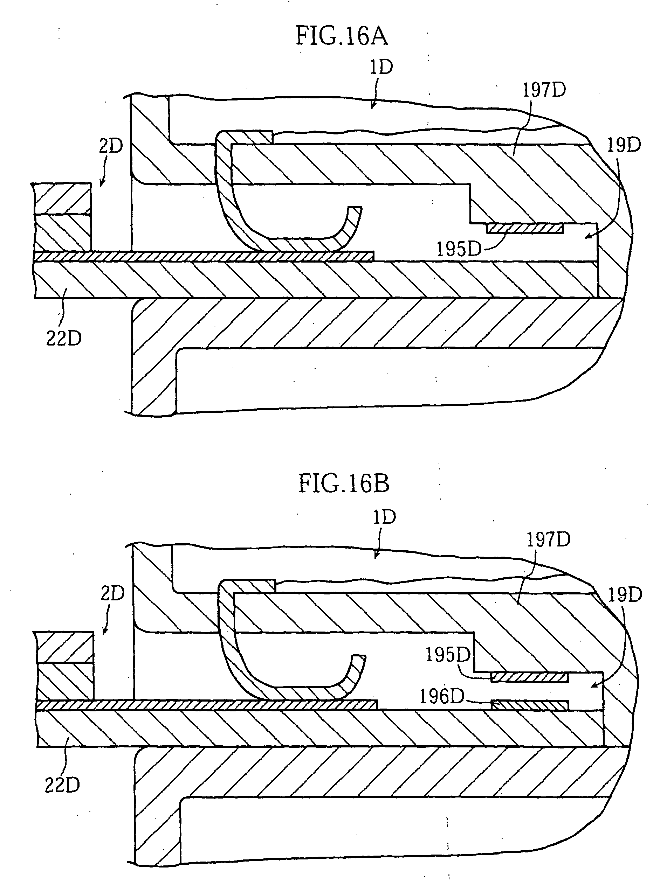

On the other hand, the analyzer 1D shown in Fig. 15 and Fig. 16 includes an information recognizer 19D for recognizing information from the electrode 196D of the biosensor 2D. The information recognizer 19D has three electrodes 195D, a capacity measurer 191D and an information calculator 192D.

Each electrode 195D of the information recognizer 19D makes a pair with a corresponding electrode 196D if the biosensor 2D has the electrode 195D. The electrode 195D is fixed in a casing 197D so as to face the corresponding electrode 196D if the biosensor 2D is formed with the electrode 196D.

The capacity measurer 191D measures a capacity of a capacitor constituted by the electrode 195D of the information recognizer 19D and the electrode 196D of the biosensor 2D.

The information calculator 192D calculates information added to the biosensor 2D, based on results of the measurements obtained by the capacity measurers 191D. Specifically, information calculator 192D checks each electrode 195D of the information recognizer 19D to see if a capacitor is constituted by insertion of the biosensor 2D, and makes calculation based on a combination pattern of the constituted capacitors, in order to obtain information added to the biosensor 2D.

Obviously, the electrode 196D in the biosensor 2D may have its area varied: By giving a small area or a large area, adjustment can be made to the size of overlapped surface made with the electrode 195D of the information recognizer 19D. In other words, by selecting a large value or a small value for the capacitor formed by the electrodes 195D, 196D, an arrangement may be made for the analyzer 1D to recognize information represented by the capacity value of the capacitor.

As expected clearly from the first through the fourth embodiments, there is no rigid limitation to locations for the first and the second electrodes of the information recognizer (capacity sensor), so they can be disposed at different places as far as the positional relationship between the electrodes can be varied by the insertion of the biosensor. Thus, freedom of designing the analyzer is not very much limited when the arrangements are made for the analyzer to be able to recognize information from the biosensor.

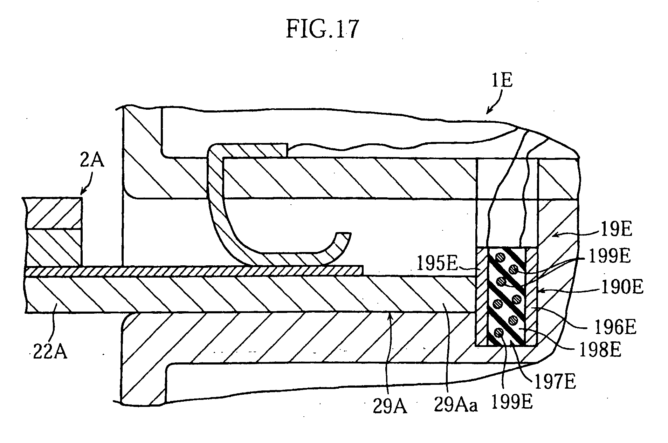

Next, a fifth embodiment of the present invention will be described with reference to Fig. 17 through Fig. 20.

A biosensor 2A according to the present embodiment is the same as the one used in the first embodiment (See Fig. 2 through Fig. 4). Specifically, the biosensor 2A includes a substrate 22A having an end which includes specific regions where projections 29Aa can be made. By selecting whether or not to form a projection 29Aa, for each of these regions, information is assigned for recognition by an analyzer 1E.

On the other hand, the analyzer 1E is basically the same as the analyzer 1A (See Fig. 1) according to the first embodiment, but differs from the analyzer 1A (See Fig. 1) according to the first embodiment in the construction of an information recognizer 19E.

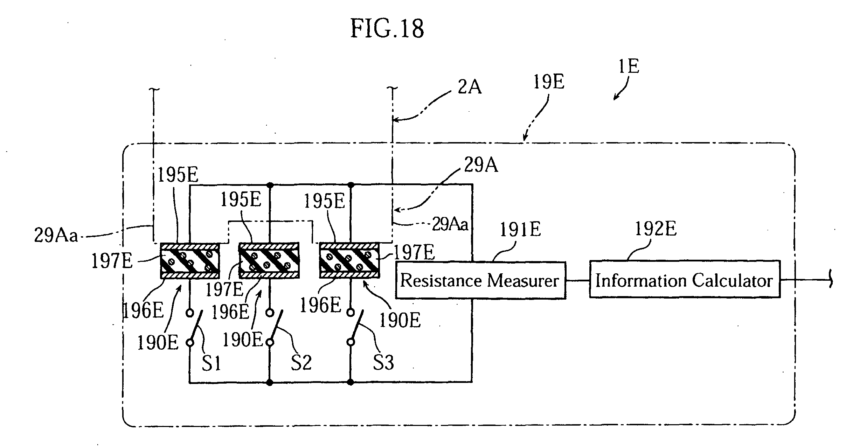

The information recognizer 19E recognizes information about e.g. the biosensor 2A, on the basis of construction of an information carrier 29A of the biosensor 2A. As shown in Fig. 18, the information recognizer 19E has three information recognition elements 190E, a resistance measurer 191E and an information calculator 192E.

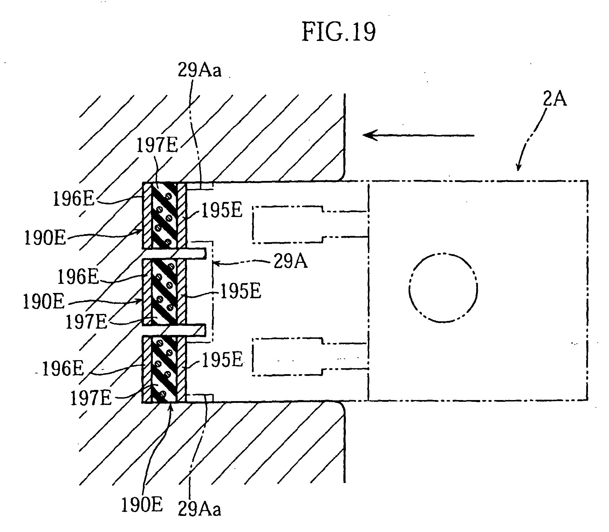

As expected from Fig. 17 and Fig. 19, each information recognition element 190E is located so that it can be pressed by a corresponding projection 29Aa of a biosensor 2A when the biosensor 2A is attached to the analyzer 1E. Each information recognition element 190E includes a first and a second electrodes 195E, 196E and a pressure sensitive electric conductor 197E sandwiched by these electrodes 195E, 196E.

The pressure sensitive electric conductor 197E is provided by a rubber member 198E serving as an elastic member, and including electrically conductive particles 199E which have an elasticity coefficient smaller than that of the rubber member 198. Further, the pressure sensitive electric conductor 197E (or the rubber member 198E more accurately) makes elastic compression when pressed by the first or the second electrodes 195E, 196E. In other words, in each information recognition element 190E, the volumetric ratio of the electrically conductive particles 199E varies in accordance with the extent of the elastic compression (volume variation), and therefore the element serves as a variable resisterwhichgives various resistance values.

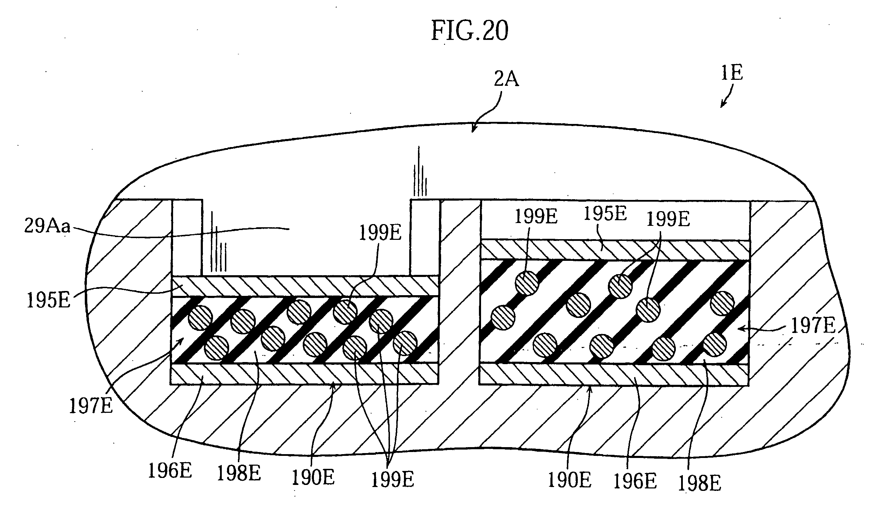

In the analyzer 1E, as shown in Fig. 20, attachment of a biosensor 2A causes the information recognition elements 190E to be pressed by corresponding projections 29Aa formed in the biosensor 2A on the side where the first electrode 195E is, thereby compressing respective pressure sensitive electric conductors 197E (rubber members 198E). This decreases the electric resistance of the pressure sensitive electric conductors 197E, and increases the electric current passing through the information recognition elements 190E.

As shown in Fig. 18, the resistance measurer 191E is connected with the first and the second electrodes 195E, 196E via switches S1-S3. Therefore, in the resistance measurer 191E can measure the resistance value of each information recognition elements 190E by selecting the open/close status of the switches S1-S3.

The information calculator 192E performs calculation to obtain information supplied from the information carrier 29A of the biosensor 2A, based on the resistance values of each information recognition element 190E. The information calculator 192E compares e.g. a result of measurement with a predetermined threshold value set for each information recognition element 190E, andusesaresult of the comparison as the basis of calculation. The threshold value is, for example, an intermediate value between a resistance value when the information recognition element 190E is pressed by the projection 29Aa of the biosensor 2A and a resistance value when it is not pressed. Then, the information recognition elements 190E pressed by the projection 29Aa of the biosensor 2A is recognized as having a smaller resistance value (L signal) than the threshold value, by the information calculator 192E due to a decreased resistance value of the pressure sensitive electric conductor 197E. On the other hand, an information recognition element 190E which is not pressed by the projection 29Aa is recognized as having a larger resistance (H signal) than the threshold value.

The biosensor 2A has up to three projections 29Aa, and the information recognizer 19E is provided with three information recognition elements 190E. This provides a total of eight combinations of the H signal and the L signal obtainable by the information recognizer 19E, and thus, the information recognizer 19E can recognize eight kinds of information individually.

Again in this embodiment, the signal can be multi-leveled. For example, the length of the projection 29Aa may be adjusted so that the information recognition element 190E will be compressed to a plurality of extents, enabling recognition of the plurality of levels with regard to the L signal.

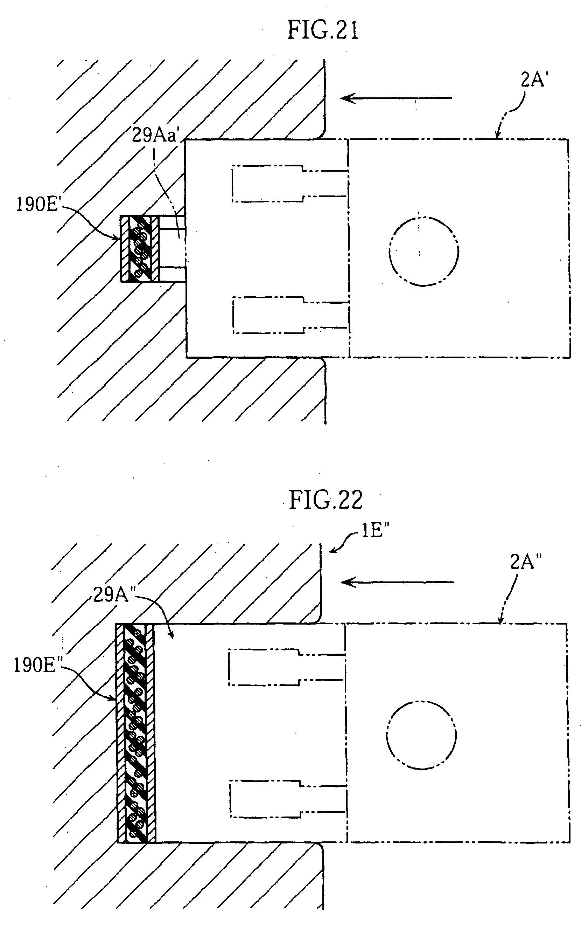

The information recognizer according to the present embodiment may be used as is the information recognizer in the first embodiment, and can be used to recognize that the biosensor has been attached for example, in addition to recognizing information relevant to the biosensor. Such a recognition of the attachment of the biosensor can be achieved as shown in Fig. 21, by providing a biosensor 2E' with an attachment recognition projection 29Aa' and providing an information recognition elements 190E' at a location corresponding to the projection 29Aa'. In this arrangement, attachment of the biosensor 2A' causes the projection 29Aa' to press the information recognition elements 190E', thereby giving an L signal, whereas an H signal is given if the biosensor 2A' is not attached. Thus, the attachment of the biosensor 2A' can be recognized when the L signal is obtained. With this arrangement, the information recognizer may turn on'the main power for example, upon reception of the L signal.

Attachment recognition and turning on of the main power such as the above are also achievable with an arrangement shown in Fig. 22. The arrangement shown in the figure uses a biosensor 2A" which is not provided with projections, and the information carrier 29A" is flat.

As alternative arrangements, in order to select the amount of compression (resistance value) of the information recognition elements 190E', 190E", adjustment may be made to the length of the projection 29Aa in the arrangement in Fig. 21 whereas the amount of insertion of the biosensor 2A" into the analyzer 1E" may be controlled in the arrangement shown in Fig. 22. In these cases, analog information is obtainable from the information recognition elements 190E', 190E".

Obviously, a plurality of information recognition elements may be placed, with one of these used for attachment recognition while the others used for recognition of information about the biosensor. For example, in the information recognizers 19E in Fig. 18, Fig. 19 and so on, one information recognition element 190E of the three information recognition elements 190E may be used as a sensor for detecting attachment of the biosensor (thereby turning on the main power), and the remaining two may be used for information recognition for selecting the calibration curve.

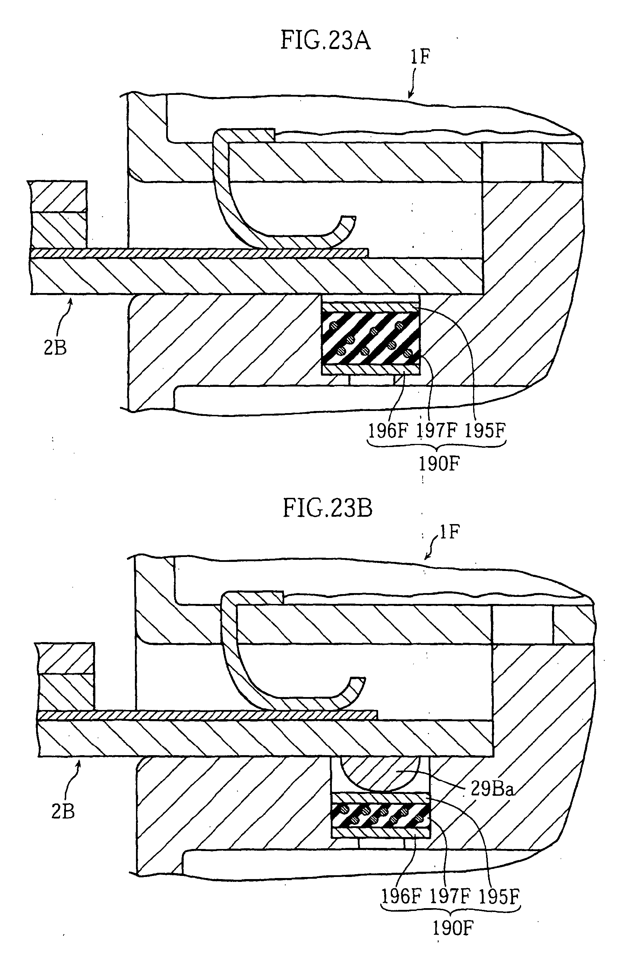

Next, a sixth embodiment will be described with reference to Fig. 23A and Fig. 23B.

The present embodiment uses the biosensor 2B which has been described with reference to Fig. 11.

On the other hand, an analyzer 1F is the analyzer 1B according to the second embodiment, with the capacity sensors 190B replaced with information recognition elements 190F which are similar to the information recognition elements 190E in the analyzer 1E according to the fifth embodiment.

Each information recognition element 190F is pressed by a corresponding projection 29Ba of the biosensor 2B when the biosensor 2B is attached to the analyzer 1F. Each information recognition element 190F includes a first and a second electrodes 195F, 196F and a pressure sensitive electric conductor 197F sandwiched by these electrodes 195F, 196F.

In this arrangement, as shown clearly in Fig. 23B, when the information recognition element 190F is pressed by the projection 29Ba of the biosensor 2B, there is a change in the distance between the first and the second electrodes 195F, 196F in directions of the thickness of the biosensor 2B. This compression varies the volume of the pressure sensitive electric conductor 197F. With such a change in the volume, the information recognition elements 190F having its volume changed outputs an L signal. On the other hand, as shown in Fig. 23A, an information recognition element 190F which is not pressed by a projection 29Bb and therefore has no change in the distance between its first and second electrodes 195A, 196A outputs an H signal.

As expected clearly from the fifth and the sixth embodiments, there is no rigid limitation to locations of the information recognition elements 190E, 190F, and they can be provided at many different places as far as their positional relationship can be varied by the insertion of the biosensor. Thus, freedom of designing the analyzer in the present invention is higher than in the conventional arrangement.

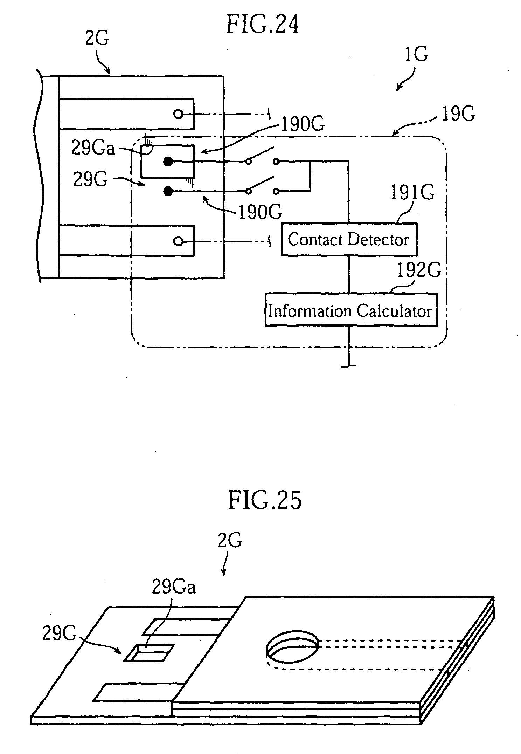

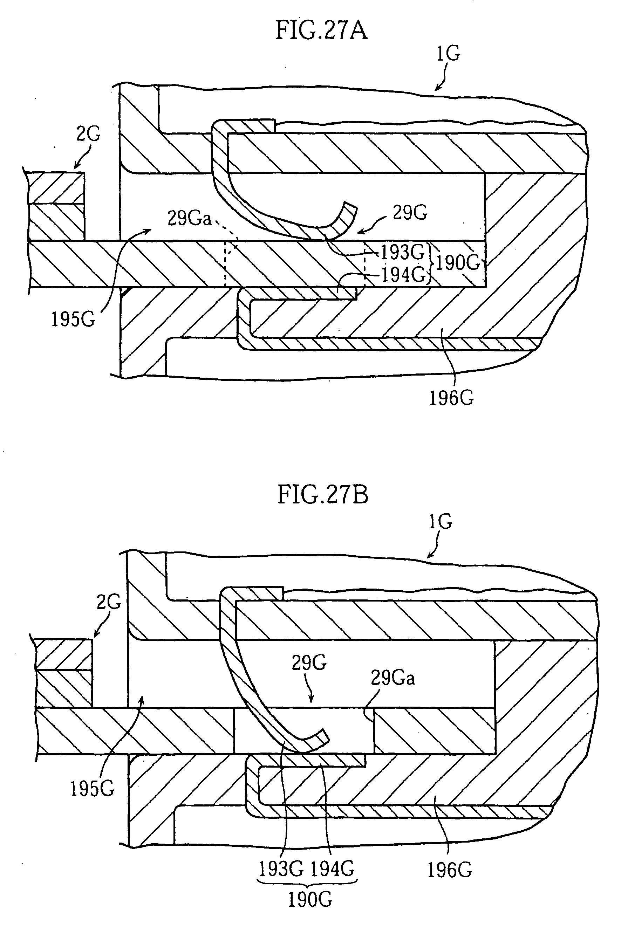

Next, a seventh embodiment of the present invention will be described with reference to Fig. 24. through Fig. 27.

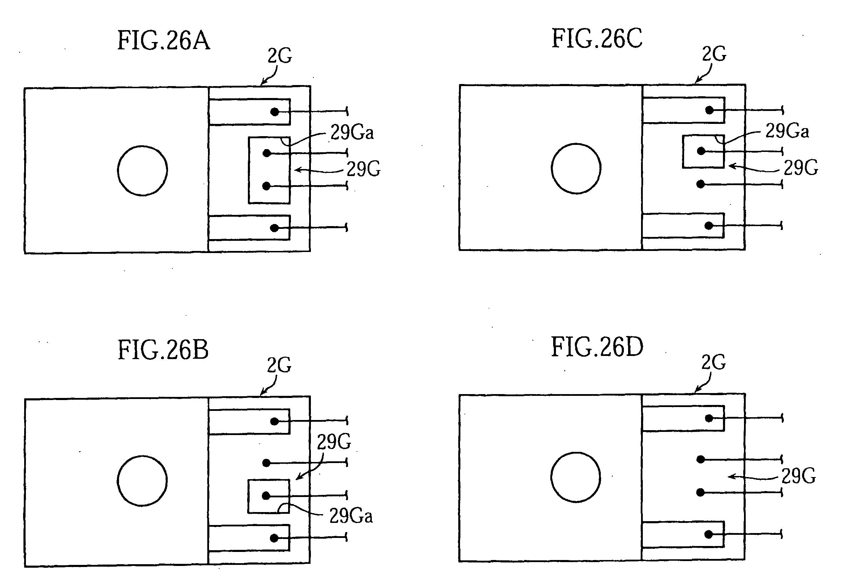

As shown in Fig. 24 and Fig. 25, a biosensor 2G has an end provided with an information carrier 29G. The information carrier 29G allows an information recognizer 19G of the analyzer 1G to recognize information about e.g. the biosensor 2G. As shown in Fig. 26A through Fig. 26D for example, the information carrier 29G has two predetermined regions. By selecting whether or not to form a through-hole 29Ga, for each of the two regions, information is assigned for recognition by the information recognizer 19G of the analyzer 1G (See Fig. 24). The information carrier 29G can be formed by punching for example, or other means which are much easier than screen printing or vapor depositing. Therefore, addition of the information carrier 29G to the biosensor 2G does not very much decrease operation efficiency.

The information recognizer 19G of the analyzer 1G in Fig. 24 recognizes information added to the biosensor 2G, on the basis of construction of the information carrier 29G of the biosensor 2G. The information recognizer 19G has two switches 190G, a contact detector 191G and an information calculator 192G.

As expected from Fig. 27, each switch 190G is located so that it can sandwich an end of the biosensor 2G when the biosensor 2G is attached to the analyzer 1G. Eachswitch 190G has a first and a second conductor members 193G, 194G. The first conductor member 193G is provided by a leaf spring, which normally makes contact with the second conductor member 194G. The second conductor member 194G is fixed to a casing 194G, to face an inside of a receptacle 195G into which the biosensor 2G is to be attached.

In the switch 190G, when the biosensor 2G has a through-hole 29Ga at a corresponding location, the first and the second conductor members 193G, 194G keep their contact via the through-hole 29Ga. On the other hand, when there is no through-hole 29Ga corresponding to the switch 190G, the end of the biosensor 2G comes between the first and the second conductor members 193G, 194G, breaking the contact between the first and the second conductor members 193G, 194G.

The contact detector 191G detects if there is a contact between the first and the second conductor members 193G, 194G for each switch 190G.

The information calculator 192G performs calculation to obtain information supplied from the information carrier 29G of the biosensor 2G, on the basis of the combination pattern of the contact/non-contact status of the switches 190G.

According to the present embodiment, up to two through-holes 29Ga can be formed in the biosensor 2G, and the information recognizer 19G is provided with two switches 190G. This provides a total of four combinations of the contact/non-contact status obtainable by the information recognizer 19G, and thus, the information recognizer 19G can recognize four kinds of information individually.

In the description of the above embodiment, the information recognizer 19G has two switches 190G. However, the number of the switches may not be two. The quantity of the switches may be selected in accordance with types of information to be recognized by the information recognizer.

In the first through the seventh embodiments so far described, the sample is analyzed in an electrochemical method. However, the present invention is also applicable to cases where analysis of the sample is made in an optical method or where analysis of the sample is made with an analyzing article which is not a biosensor.

## Claims

1. An information recognizing analyzer used with an analyzing article attached thereto, for analysis of a specific component in a sample liquid supplied to the analyzing article,. comprising:
an information recognizer for recognition of information added to the analyzing article,
wherein the information recognizer includes an electro-physical-quantity variable part which has different electro-physical quantities in accordance with the information added to the analyzing article, upon attachment of the analyzing article.

2. The information recognizing analyzer according to Claim 1, wherein the electro-physical-quantity variable part includes a pair of a first electrode and a second electrode in a relative positional relationship variable upon attachment of the analyzing article.

3. The information recognizing analyzer according to Claim 2, wherein the first electrode and the second electrode have their distance between the two varied.

4. The information recognizing analyzer according to Claim 3, wherein at least one of the first electrode and the second electrode in the information recognizer further includes a fixed elastic member,
the distance between the first electrode and the second electrode being varied by an elastic deformation of the elastic member.

5. The information recognizing analyzer according to Claim 2, wherein the first electrode and the second electrode have their area of mutually opposed surfaces varied.

6. The information recognizing analyzer according to Claim 5, wherein at least one of the first electrode and the second electrode in the information recognizer moves upon attachment of the analyzing article, in a direction of insertion of the analyzing article.

7. The information recognizing analyzer according to Claim 1, wherein the information recognizer includes pairs of electrodes each provided by a first electrode and a second electrode in a relative positional relationship variable upon attachment of the analyzing article,
information being recognized individually from each pair of electrodes.

8. The information recognizing analyzer according to Claim 2, wherein the information recognizer further includes:
a capacity measurer for measurement of a capacity of a capacitor constituted by the first electrode and the second electrode; and
an information calculator for recognition of information added to the analyzing article, based upon a comparison between a result of measurement obtained by the capacity measurer and a predetermined threshold value.

9. The information recognizing analyzer according to Claim 1, wherein the electro-physical-quantity variable part includes a pressure sensitive electric conductor having a resistance value variable upon attachment of the analyzing article.

10. The information recognizing analyzer according to Claim 1, wherein the electro-physical-quantity variable part includes a plurality of pressure sensitive electric conductors having a resistance value variable upon attachment of the analyzing article,
information being recognized individually from each pressure sensitive electric conductor.

11. The information recognizing analyzer according to Claim 9, wherein the information recognizer further includes:
a resistance value measurer for measurement of a resistance value of the pressure sensitive electric conductor; and
an information calculator for recognition of information added to the analyzing article, based upon a comparison between a result of measurement obtained by the resistance value measurer and a predetermined threshold value.

12. An information recognizing analyzer used with an analyzing article attached thereto, for analysis of a specific component in a sample liquid supplied to the analyzing article, comprising:
an information recognizer for recognition of information added to the analyzing article,
wherein the information recognizer includes a first and a second conductors,
the first and the second conductors being selectively brought into or out of mutual contact in accordance with the information added to the analyzing article, upon attachment of the analyzing article.

13. The information recognizing analyzer according to Claim 12, wherein the information recognizer further includes:
a contact detector for detection of a contact between the first conductor and the second conductor; and
an information calculator for recognition of information added to the analyzing article, based upon a result of detection obtained by the contact detector.

14. An analyzing article comprising an information carrier for giving information to an analyzer which includes an information recognizer for recognition of the information carried by the information carrier, the analyzing article being used as attached to the analyzer,
the information recognizer including an electro-physical-quantity variable part having different electro-physical quantities upon attachment of the analyzing article,
wherein the information carrier is provided by a projection or a hole related to the information to be recognized by the analyzer.

15. The analyzing article according to Claim 14, formed essentially into a platy shape, the projection or the hole projecting or recessing in a direction generally perpendicular to a thickness direction of the analyzing article.

16. The analyzing article according to Claim 14, formed essentially into a platy shape, the projection or the hole projecting or recessing in a thickness direction of the analyzing article.

17. The analyzing article according to Claim 14, wherein the projection or the hole projects or recesses by an amount related to the information to be recognized by the analyzer.

18. The analyzing article according to Claim 15, wherein the hole is provided by a through-hole.

19. A unit of an analyzing article and an analyzer for analysis of a specific component in a sample liquid supplied to the analyzing article,
wherein the analyzer includes a first electrode fixed therein,
the analyzing article including a second electrode fixed therein so as to face the first electrode and thereby constitute a capacitor upon attachment of the analyzing article to the analyzer.

20. The unit according to Claim 19, wherein the analyzer includes:
a capacity measurer for measurement of a capacity of the capacitor constituted by the first electrode and the second electrode; and
an information calculator for recognition of information added to the analyzing article, based upon a comparison between a result of measurement obtained by the capacity measurer and a predetermined threshold value.
